# EUROPEAN PATENT APPLICATION

(11) **EP 3 919 125 A1**
(43) Date of publication of application: **08.12.2021**
(21) Application number: 20747826.4
(22) Date of filing: 16.01.2020
(51) Int. Cl.: A61P 25/20, A61P 25/28, A61P 43/00, A23L 29/00, A23L 33/10, A61K 31/4745

(54) **COMPOSITION FOR COMPETITIVE INHIBITION OF OREXIN RECEPTORS**

(30) Priority: 28.01.2019 JP 2019011849
(71) Applicant: Suntory Holdings Limited, Osaka 530-8203 (JP)
(72) Inventor: ITOGA, Shota, Soraku-gun, Kyoto 619-0284 (JP); WATANABE, Hiroshi, Soraku-gun, Kyoto 619-0284 (JP); TAKANO, Jiro, Soraku-gun, Kyoto 619-0284 (JP)
(74) Representative: Vossius & Partner Patentanwälte Rechtsanwälte mbB
(86) International application number: PCT/JP2020/001288
(87) International publication number: WO 2020/158415

(57) **Abstract**

The present invention aims to provide a novel composition for competitively inhibiting an orexin receptor, which can inhibit binding of orexin to its receptor, and a method of inhibiting binding of orexin to its receptor. The present invention relates to, for example, a composition for competitively inhibiting an orexin receptor, containing pyrroloquinoline quinone or a salt thereof as an active ingredient.

## Description

### TECHNICAL FIELD

The present invention relates to a composition for competitively inhibiting an orexin receptor. The present invention also relates to a method of inhibiting binding of orexin to its receptor (orexin receptor) and the like.

### BACKGROUND ART

Orexin is a neuropeptide produced by nerve cells present in the lateral hypothalamic area. Orexin receptors are orexin-specific receptors. Actions such as increase in wakefulness level and increase in food intake in animals are exerted in response to action of orexin on an orexin receptor. Thus, a substance having an action to inhibit binding of orexin to its receptor (orexin receptor competitive inhibition action) is expected to act to inhibit wakefulness or the like.

Patent Literature 1 discloses a compound used as an orexin receptor antagonist. Known orexin receptor competitive inhibitors include suvorexant. Yet, no naturally occurring compounds are known which have orexin receptor competitive inhibition action.

### CITATION LIST

### - Patent Literature

Patent Literature 1: JP 2018-135349 A

### SUMMARY OF INVENTION

### - Technical Problem

The present invention aims to provide a novel composition for competitively inhibiting an orexin receptor, which can inhibit binding of orexin to its receptor. The present invention also aims to provide a method of inhibiting binding of orexin to its receptor.

### - Solution to Problem

As a result of extensive studies to solve the above issue, the present inventors found that pyrroloquinoline quinone or a salt thereof has excellent orexin receptor competitive inhibition action. Pyrroloquinoline quinone is a compound found in various edible plants, and is considered to be highly safe for long-term intake.

Specifically, the present invention relates to the following composition for competitively inhibiting an orexin receptor and the like.
(1) A composition for competitively inhibiting an orexin receptor, containing pyrroloquinoline quinone or a salt thereof as an active ingredient.
(2) The composition for competitively inhibiting an orexin receptor according to (1) above, wherein the salt of pyrroloquinoline quinone is a sodium salt of pyrroloquinoline quinone.
(3) The composition for competitively inhibiting an orexin receptor according to (1) or (2) above, wherein the composition is a food or beverage.
(4) The composition for competitively inhibiting an orexin receptor according to any one of (1) to (3) above, wherein the composition is labeled with a function claim based on orexin receptor antagonist action.
(5) A method of inhibiting binding of orexin to its receptor, including administering pyrroloquinoline quinone or a salt thereof.
(6) Use of pyrroloquinoline quinone or a salt thereof for inhibiting binding of orexin to its receptor.

### - Advantageous Effects of Invention

The present invention can provide a novel composition for competitively inhibiting an orexin receptor, which can inhibit binding of orexin to its receptor. The composition for competitively inhibiting an orexin receptor of the present invention, which contains, as an active ingredient, a highly safe substance having excellent orexin receptor competitive inhibition action, is useful as a food or beverage, a pharmaceutical product, or the like. The present invention also provides a method of inhibiting binding of orexin to its receptor.

### BRIEF DESCRIPTION OF DRAWINGS

Fig. 1 is a graph showing enzyme activity in negative control group (N.C.), positive control group (P.C.), and pyrroloquinoline quinone disodium salt-added group (PQQ).
Fig. 2 is a graph showing dehydrogenase activity in the negative control group (N.C.), the positive control group (P.C.), and the pyrroloquinoline quinone disodium salt-added group (PQQ).

### DESCRIPTION OF EMBODIMENTS

The composition for competitively inhibiting an orexin receptor of the present invention contains pyrroloquinoline quinone or a salt thereof as an active ingredient.

Two types of orexin, orexin-A and orexin-B, have been identified. These orexins are produced in the lateral hypothalamic area. There are two subtypes of orexin receptor, i.e., orexin-1 receptor and orexin-2 receptor. The orexin receptor in the present invention encompasses both subtypes, with the orexin-2 receptor being preferred. In one embodiment, the composition for competitively inhibiting an orexin receptor of the present invention is suitably used to inhibit binding of orexin-A to the orexin-2 receptor.

Pyrroloquinoline quinone is a redox coenzyme. Pyrroloquinoline quinone or a salt thereof is found in various organisms including plants, bacteria, and animals, and thus can be prepared by extraction from various organisms. Commercially available pyrroloquinoline quinone or a salt thereof can also be used. In the present invention, the composition of the present invention may contain a raw material derived from plants rich in pyrroloquinoline quinone or a salt thereof, for example, as long as the effect of the present invention is achieved.

The salt of pyrroloquinoline quinone is preferably a salt that can be used in a food or beverage, pharmaceutical product, or the like. Examples include alkali metal salts such as a sodium salt and a potassium salt, and alkaline earth metal salts such as a calcium salt and a magnesium salt. The salt is preferably a sodium salt.

The number of substituted atoms in the salt of pyrroloquinoline quinone is 1 to 3, preferably 2. One type or two or more types of the salt of pyrroloquinoline quinone may be used. Preferably, the salt of pyrroloquinoline quinone is a disodium salt of pyrroloquinoline quinone.

As shown in Examples described later, pyrroloquinoline quinone or a salt thereof has an action to inhibit binding orexin to its receptor. Thus, pyrroloquinoline quinone or a salt thereof acts as an orexin receptor antagonist. Pyrroloquinoline quinone or a salt thereof can be used to inhibit binding of orexin to its receptor in a subject.

Pyrroloquinoline quinone or a salt thereof is a compound that is contained in natural products, foods, and beverages and that has been used as a food ingredient. Thus, daily intake of pyrroloquinoline quinone or a salt thereof, for example, is considered to be unlikely to cause problems in terms of safety. The present invention can provide a composition for competitively inhibiting an orexin receptor, which contains a highly safe component as an active ingredient.

The composition for competitively inhibiting an orexin receptor of the present invention is applicable for therapeutic use (medical use) or non-therapeutic use (non-medical use). The "non-therapeutic" is a concept that does not include medical activities, i.e., a concept that does not include methods of surgery, therapy, or diagnosis of humans.

The composition for competitively inhibiting an orexin receptor of the present invention can be provided in the form of a food or beverage, pharmaceutical or quasi-pharmaceutical product, feed, or the like. The composition for competitively inhibiting an orexin receptor of the present invention may be a food or beverage, pharmaceutical or quasi-pharmaceutical product, feed, or the like by itself for competitively inhibiting an orexin receptor, or may be a material, a preparation, or the like that is added thereto.

For example, the composition for competitively inhibiting an orexin receptor of the present invention may be provided as an agent or the like, but it is not limited thereto. The agent can be directly provided as a composition, or can be provided as a composition containing the agent. In one embodiment, the composition for competitively inhibiting an orexin receptor of the present invention can be regarded as an orexin receptor competitive inhibitor.

In order to sufficiently obtain the effect of the present invention, preferably, the composition for competitively inhibiting an orexin receptor of the present invention is a composition for oral intake. The composition for oral intake may be a food or beverage, pharmaceutical or quasi-pharmaceutical product for oral administration, or feed, preferably a food or beverage or pharmaceutical product for oral administration, still more preferably a food or beverage.

The composition for competitively inhibiting an orexin receptor of the present invention may contain optional additives and optional components, in addition to the active ingredient (pyrroloquinoline quinone or a salt thereof) used in the present invention, as long as the effect of the present invention is not impaired. Such additives and components can be selected according to the composition form or the like. Those that can be used generally in foods, beverages, pharmaceutical products, quasi-pharmaceutical products, feed, and the like can be used. When the composition for competitively inhibiting an orexin receptor of the present invention is provided as a food or beverage, pharmaceutical or quasi-pharmaceutical product, feed, or the like, any common method can be used for production.

For example, when the composition for competitively inhibiting an orexin receptor of the present invention is provided as a food or beverage, a component usable in foods or beverages (e.g., a food material or an optional food additive) can be added to the active ingredient used in the present invention to provide various types of foods or beverages. Non-limiting examples of the foods or beverages include general foods and beverages, health foods, health beverages, foods with function claims, foods for specified health uses, and foods and beverages for the sick. The health foods, the foods with function claims, the foods for specified health uses, and the like can be used, for example, in various forms of preparations such as fine granules, tablets, granules, powders, capsules, chewable tablets, syrups, liquids, and liquid foods.

When the composition for competitively inhibiting an orexin receptor of the present invention is provided as a pharmaceutical or quasi-pharmaceutical product, for example, a pharmacologically acceptable carrier, an optional additive, or the like can be added to the active ingredient used in the present invention to provide various dosage forms of pharmaceutical or quasi-pharmaceutical products. Such a carrier, an additive, or the like may be any pharmacologically acceptable one that can be used in pharmaceutical or quasi-pharmaceutical products. Examples thereof include excipients, binders, disintegrants, lubricants, antioxidants, and colorants. One or more of these can be used. The form of administration (intake) of the pharmaceutical or quasi-pharmaceutical product may be an oral or parenteral (transdermal, transmucosal, or enteral administration, injection, or the like). Oral administration is preferred in order to more sufficiently obtain the effect of the present invention. When the composition for competitively inhibiting an orexin receptor of the present invention is provided as a pharmaceutical or quasi-pharmaceutical product, preferably, it is a pharmaceutical or quasi-pharmaceutical product for oral administration. Examples of the dosage form of preparations for oral administration include liquids, tablets, powders, fine granules, granules, sugar-coated tablets, capsules, suspensions, emulsions, and chewable tablets. The pharmaceutical product may be for non-human animals.

Providing the composition for competitively inhibiting an orexin receptor of the present invention as feed only requires adding the active ingredient used in the present invention to feed. The feed includes feed additives. Examples of the feed include livestock feed for animals such as cows, pigs, chickens, sheep, and horses; feed for small animals such as rabbits, rats, and mice; and pet food for animals such as dogs, cats, and birds.

The amount of pyrroloquinoline quinone or a salt thereof in the composition for competitively inhibiting an orexin receptor of the present invention is not limited, and can be set according to the composition form and the like.

The amount of pyrroloquinoline quinone or a salt thereof, in terms of pyrroloquinoline quinone, in the composition for competitively inhibiting an orexin receptor of the present invention is preferably 0.0001 wt% or more, more preferably 0.001 wt% or more, and is preferably 90 wt% or less, more preferably 50 wt% or less, for example. In one embodiment, the amount of pyrroloquinoline quinone or a salt thereof, in terms of pyrroloquinoline quinone, in the composition for competitively inhibiting an orexin receptor is preferably 0.0001 to 90 wt%, more preferably 0.001 to 50 wt%. In one embodiment, the total amount of pyrroloquinoline quinone or a salt thereof in the composition for competitively inhibiting an orexin receptor may be 0.0001 to 97 wt% or may be 0.001 to 55 wt%. In one embodiment, when the composition for competitively inhibiting an orexin receptor of the present invention is provided as a food or beverage, pharmaceutical or quasi-pharmaceutical product, feed, or the like, preferably, the amount of pyrroloquinoline quinone or a salt thereof is in the above ranges.

The composition for competitively inhibiting an orexin receptor of the present invention can be fed or administered by a method appropriate to the composition form. Preferably, the composition for competitively inhibiting an orexin receptor of the present invention is orally fed (orally administered), in order to sufficiently obtain the effect of the present invention.

The intake (or "dosage") of the composition for competitively inhibiting an orexin receptor of the present invention is not limited, as long as it is the amount (effective amount) that provides the effect of inhibiting binding of orexin to its receptor. The amount may be suitably set according to the dosage form and administration method.

In one embodiment, when the composition for competitively inhibiting an orexin receptor is orally fed or administered to a human (adult), the intake of the pyrroloquinoline quinone or a salt thereof in terms of pyrroloquinoline quinone is preferably 1 to 200 mg, more preferably 5 to 60 mg, per 60 kg per day. Intake of the above amount in one or more portions per day, for example, one to several portions (e.g., two to three portions) per day, is preferred.

The composition for competitively inhibiting an orexin receptor of the present invention is useful in preventing or ameliorating a symptom or disease that is expected to be prevented or ameliorated by inhibiting binding of orexin to its receptor. Orexin is known to be related to sleep and wakefulness, eating behavior, and emotion. Orexin has also been reported to be involved in symptoms or diseases such as insomnia, hyperglycemia, and Alzheimer's disease. For example, intake of suvorexant, which is an orexin receptor competitive inhibitor, has been reported to have decreased sleep latency and objective wake after sleep onset and have increased total sleep duration and sleep efficiency (Sleep. 2013 Feb 1; 36(2):259-267). Suvorexant is also known to have an effect of suppressing the rise in blood sugar levels during glucose tolerance test (Endocrinology. 2016 Nov; 157(11):4146-4157. Epub 2016 Sep 15). It has also been reported that mice show an increase in amyloid β level, due to lack of sleep and injection of orexin peptide (Science. 2009 Nov 13; 326(5955):1005-1007). Meanwhile, it has been reported that amyloid precursor protein transgenic mice show a decrease in amyloid β level and in amyloid plaque formation, due to injection of almorexant, which is an orexin receptor antagonist. In addition, it has been suggested that competitive inhibition of an orexin receptor may result in anti-obesity, appetite suppression, anti-depression, and/or anxiolytic effect. Thus, intake or administration of the composition for competitively inhibiting an orexin receptor of the present invention is expected to provide effects such as improved sleep (for example, sleep quality improvement such as decreased sleep latency, decreased wake after sleep onset, and decreased number of wake after sleep onset), prevention or amelioration of abnormal blood sugar levels, and prevention or amelioration of Alzheimer's disease, due to the orexin receptor competitive inhibition action. Improved sleep can, for example, prevent or ameliorate sleep disorders.

Herein, prevention of a symptom or disease includes preventing the onset of a symptom or disease, delaying the onset of a symptom or disease, reducing the incidence of a symptom or disease, reducing the onset risk of a symptom or disease, and the like. Amelioration of a symptom or disease includes helping a subject recover from a symptom or disease, alleviating a symptom or disease, delaying or preventing symptom exacerbation or disease progression, and the like. Recovery includes at least partial recovery.

A subject (or "subject for administration") to be fed or administered with the composition for competitively inhibiting an orexin receptor of the present invention is not limited. The subject is preferably a human or non-human mammal, more preferably a human.

Preferably, the subject to be fed or administered with the composition for competitively inhibiting an orexin receptor of the present invention is one who wants or needs to inhibit binding of orexin to its receptor. Examples of the subject include humans with symptoms such as insomnia, hyperglycemia, Alzheimer's disease, or the like. The composition for competitively inhibiting an orexin receptor of the present invention can also be used, for example, for a healthy person for purposes such as prevention of a symptom or disease that is expected to be prevented or ameliorated by inhibiting binding of orexin to its receptor.

The composition for competitively inhibiting an orexin receptor of the present invention may be labeled with a function claim based on the orexin receptor competitive inhibition action. The composition for competitively inhibiting an orexin receptor of the present invention may be labeled with one or more function claims, such as "improving sleep", "improving blood sugar levels", "preventing cognitive decline", and the like.

In one embodiment of the present invention, preferably, the composition for competitively inhibiting an orexin receptor of the present invention is a food or beverage labeled with one or more function claims described above. The labels indicating the above function claims may be labels indicating use for achieving these functions.

The present invention encompasses the following method and use:
a method of inhibiting binding of orexin to its receptor, including administering pyrroloquinoline quinone or a salt thereof; and
use of pyrroloquinoline quinone or a salt thereof for inhibiting binding of orexin to its receptor.

The method and the use may be therapeutic or non-therapeutic. Administering (or feeding) pyrroloquinoline quinone or a salt thereof to a subject can inhibit binding of orexin to its receptor (orexin receptor) in the subject. This action is expected to produce effects such as improved sleep, improve blood sugar levels, and prevention of Alzheimer's disease. In one embodiment, pyrroloquinoline quinone or a salt thereof can be used to prevent or ameliorate sleep disorders by inhibiting binding of orexin to its receptor.

In the above method and use, pyrroloquinoline quinone or a salt thereof and preferred embodiments thereof are as described above for the composition for competitively inhibiting an orexin receptor of the present invention.

The above method and use may only require administering pyrroloquinoline quinone or a salt thereof to a subject at least once a day, for example, once to several times (for example, two or three times) a day.

The above use is preferably for humans or non-human mammals, more preferably for humans.

The above method and use may only require administering a subject with pyrroloquinoline quinone or a salt thereof in an amount (or "effective amount") capable of achieving the orexin receptor competitive inhibition action. A preferred dosage of pyrroloquinoline quinone or a salt thereof, a preferred subject for administration, a preferred administration method, and the like are as described above for the composition for competitively inhibiting an orexin receptor of the present invention. Pyrroloquinoline quinone or a salt thereof may be administered directly or may be administered as a composition containing pyrroloquinoline quinone or a salt thereof. For example, the composition for competitively inhibiting an orexin receptor of the present invention described above may be administered.

Pyrroloquinoline quinone or a salt thereof can be used for producing a food or beverage, pharmaceutical or quasi-pharmaceutical product, feed, or the like used for inhibiting binding of orexin to its receptor. In one embodiment, the present invention also encompasses use of pyrroloquinoline quinone or a salt thereof for producing a composition for competitively inhibiting an orexin receptor.

### EXAMPLES

The following provides examples that more specifically describe the present invention, but the present invention is not limited to these examples.

### <Example 1>

The orexin receptor antagonist activity of a test substance was measured using an accessory to Orexin 2 Receptor Reporter Assay Kit (Cayman Chemical Company).

In this evaluation system, cells that express orexin receptors at cell surfaces are used. Binding of the agonist (orexin) to the orexin receptor causes secretion of secreted alkaline phosphatase (SEAP). Secreted SEAP is reacted with an alkaline phosphatase substrate, and the enzyme activity of SEAP is measured using a luminometer. A substance having orexin receptor antagonist action reduces the enzyme activity.

(1) HEK293T cells were prepared at a concentration of 2.5 × 10⁵ cells/mL using Dulbecco's modified Eagle medium (high glucose content) (Dulbecco's Modified Eagle's Medium-high glucose, with 4500 mg/L glucose, L-glutamine, sodium pyruvate, and sodium bicarbonate, liquid, sterile-filtered, suitable for cell culture) (Sigma-Aldrich) to which fetal bovine serum (FBS) was added to a final concentration of 10%.
(2) The medium containing HEK293T cells prepared in (1) was seeded at 200 µL per well in Orexin 2 Receptor Reverse Transfection Strip Plate.
(3) Incubation was performed at 5% CO₂ and 37°C for 16 hours.
(4) After removal of the supernatant, pyrroloquinoline quinone disodium salt (Mitsubishi Gas Chemical Company, Inc.) was added to a final concentration of 100 µg/mL, followed by incubation at 5% CO₂ and 37°C for one hour. The solvent used was Dulbecco's modified Eagle medium (high glucose content) (Dulbecco' s Modified Eagle' s Medium-high glucose, with 4500 mg/L glucose, L-glutamine, sodium pyruvate, and sodium bicarbonate, liquid, sterile-filtered, suitable for cell culture) (Sigma-Aldrich).
(5) Orexin-A was added to a final concentration of 50 nM, followed by incubation at 5% CO₂ and 37°C for 24 hours.
(6) The supernatant was collected.
(7) The enzyme (SEAP) activity of the supernatant was measured in accordance with a protocol attached to Orexin 2 Receptor Reporter Assay Kit (Cayman Chemical Company).
(8) In this test, a group to which suvorexant 1 µM was added instead of pyrroloquinoline quinone disodium salt was used as positive control; and a group to which dimethyl sulfoxide (DMSO) was added instead of pyrroloquinoline quinone disodium salt was used as negative control. The test was performed with n = 3 for each group.

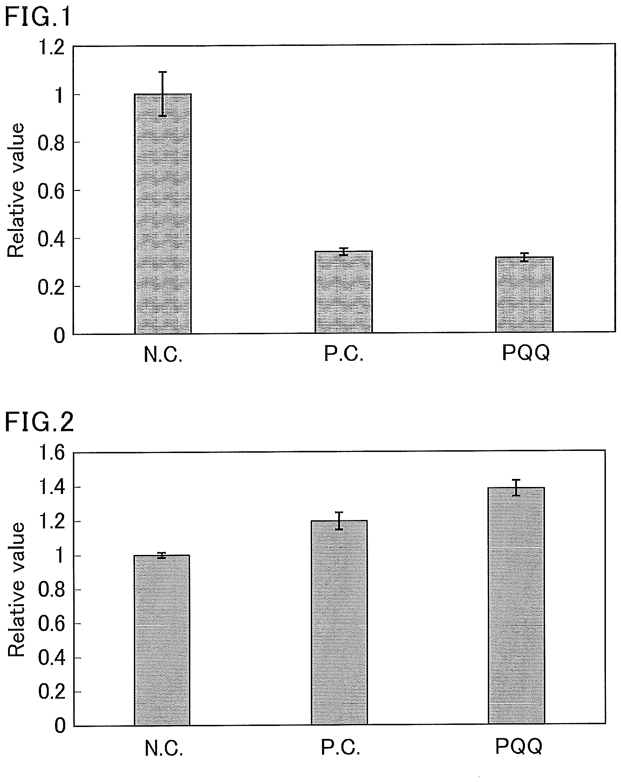

Fig. 1 shows results (average ± standard error) of the enzyme (SEAP) activity measured by the above method, as an index of the orexin receptor antagonist action. Fig. 1 is a graph showing enzyme activity in the negative control group (N.C.), the positive control group (P.C.), and the pyrroloquinoline quinone disodium salt-added group (PQQ). The results shown in Fig. 1 are expressed in values relative to the enzyme activity of the negative control group taken as 1.

In this evaluation system, the higher the level of the orexin receptor antagonist action is, the lower the enzyme activity becomes.

As shown in Fig. 1, adding 100 pg/mL pyrroloquinoline quinone disodium salt resulted in orexin receptor antagonist action equivalent to that exerted by addition of 1 µM suvorexant.

### <Example 2>

For the pyrroloquinoline quinone disodium salt-added group in Example 1, WST-8 Cell Proliferation Assay Kit (Cayman Chemical Company) was used to confirm the cell growth after addition of pyrroloquinoline quinone disodium salt. The measurement was performed by a method according to an attached protocol. The dehydrogenase activity is known to be in proportion to the number of live cells. The kit utilizes this principle and measures the dehydrogenase activity released in the medium from the cells. The negative control group and the positive control group in Example 1 were also subjected to measurement of cell growth after addition of DMSO or suvorexant by the same method.

Fig. 2 is a graph showing the dehydrogenase activity in the negative control group (N.C.), the positive control group (P.C.), and the pyrroloquinoline quinone disodium salt-added group (PQQ). The results (average ± standard error) are expressed in values relative to the dehydrogenase activity of the negative control group taken as 1.

As shown in Fig. 2, the pyrroloquinoline quinone disodium salt-added group did not show a decrease in the number of cells, as compared to the positive control and the negative control. Based on the above, the decrease in enzyme activity due to addition of a pyrroloquinoline quinone disodium salt, which was observed in Example 1, is presumably caused by the antagonist action on the orexin receptor but not by cell death.

## Claims

1. A composition for competitively inhibiting an orexin receptor, comprising:
pyrroloquinoline quinone or a salt thereof as an active ingredient.

2. The composition for competitively inhibiting an orexin receptor according to claim 1,
wherein the salt of pyrroloquinoline quinone is a sodium salt of pyrroloquinoline quinone.

3. The composition for competitively inhibiting an orexin receptor according to claim 1 or 2,
wherein the composition is a food or beverage.

4. The composition for competitively inhibiting an orexin receptor according to any one of claims 1 to 3,
wherein the composition is labeled with a function claim based on orexin receptor antagonist action.

5. A method of inhibiting binding of orexin to its receptor, comprising:
administering pyrroloquinoline quinone or a salt thereof.

6. Use of pyrroloquinoline quinone or a salt thereof for inhibiting binding of orexin to its receptor.
